Europäisches Patentamt

European Patent Office

Office européen des brevets

(11) Publication number: **0 388 125**
**A1**

## (12) EUROPEAN PATENT APPLICATION

(21) Application number: **90302615.1**

(22) Date of filing: **12.03.90**

(51) Int. Cl.⁵: **A61K 31/485, //(A61K31/485, 31:19)**

(30) Priority: **14.03.89 GB 8905815**

(43) Date of publication of application:
**19.09.90 Bulletin 90/38**

(84) Designated Contracting States:
**BE DE ES FR GB IT NL SE**

(71) Applicant: **BEECHAM GROUP PLC**
**Beecham House Great West Road**
**Brentford Middlesex TW8 9BD(GB)**

(72) Inventor: **Davis, Adrian Francis, Beecham Products**
**Research Department, St. George's Avenue**
**Weybridge, Surrey, RH3 0DE(GB)**
Inventor: **James, Kenneth William, Beecham Products**
**Research Department, St. George's Avenue**
**Weybridge, Surrey, RH3 0DE(GB)**

(74) Representative: **Lockwood, Barbara Ann et al**
**Smithkline Beecham, Corporate Patents,**
**Great Burgh, Yew Tree Bottom Road**
**Epsom, Surrey KT18 5XQ(GB)**

(54) **Medicament.**

(57) A pharmaceutical composition comprising from 100 to 600mg of ibuprofen (expressed as the weight of the free acid) and from 12 to 40mg of codeine (expressed as the weight of the free base) or pharmaceutically acceptable salts thereof is effective for the relief of acute pain in adult humans after the administration of only a single dose.

EP 0 388 125 A1

## MEDICAMENT

The present invention relates to the use of a combination of ibuprofen and codeine in the manufacture of a medicament for the treatment of acute pain, to a novel composition thereof and its use in the treatment of acute pain.

Ibuprofen [α-methyl-4-(2-methylpropyl)benzene acetic acid] is a well established peripherally acting non-steroidal anti-inflammatory agent which also shows analgesic properties in a variety of pain states.

Codeine is a centrally acting analgesic, clinically effective on multiple dosing but showing little if any efficacy in a single dose of up to 60mg (of the phosphate salt) (Honig S, Murray K.A., J. Clin. Pharmacol.; 1984; 24; 96-102). Codeine is metabolised to morphine and this is thought to be responsible for, and may also account for the apparent delayed onset of, analgesic activity.

Combinations of ibuprofen and codeine have previously been proposed for use in the treatment of pain, for instance pain following dental surgery, on the basis that a combination would have a greater analgesic effect than either alone, because of their different modes of action. Thus, a combination of ibuprofen (400mg) and codeine phosphate (30mg), given after dental surgery was found to be effective (Giles A.D., Pickvance N.J.; Clinical Trials Journal; 1985; 22 (4); 300-312), but only after multiple doses of the combination had been administered.

In addition, whilst it has been shown that ibuprofen (400mg) in combination with codeine phosphate, when given as a single dose, is more effective in the relief of pain following dental surgery than the same amount of ibuprofen when used alone, (Copper S.A. et al, Pharmacotherapy; 1982, 2; 162-7), the amount of codeine phosphate required was 60mg, that is twice the quantity used in a multiple dosing regime.

Of the many forms in which pain is manifested, a particularly important form is acute pain, for instance, headaches, which occur not infrequently and to a wide range of the population and where fast relief of pain is very desirable. There exists a continuing need for a medicament which is not only effective in providing rapid relief following a single dose but which can also be purchased by the consumer as an over-the-counter (OTC) medicine.

Because of the recognised potential for inducing dependance liability, the amount of codeine that can be incorporated into an OTC medicine in the United Kingdom is limited, by Schedule I, Part II of the Medicines (Products other than Veterinary Drugs) (Prescription only) Order 1983, to 20mg per unit dose. In addition, the amount of ibuprofen that can be incorporated into an OTC medicine in the United Kingdom is limited to 400mg per unit dose by Schedule I, Part I of the Medicines (Products other than Veterinary Drugs) (Prescription only) Order as amended by the Medicines (Products other than Veterinary Drugs) (Prescription Only) Amendment Order 1988.

Surprisingly it has now been found that certain mixtures of ibuprofen and codeine in which the codeine is present in a relatively small amount, compared to ibuprofen, provide effective relief of pain after a single dose of medicament and are potentially useful in the treatment of acute pain.

Accordingly the present invention provides the use of ibuprofen or a pharmaceutically acceptable salt thereof; and codeine or a pharmaceutically acceptable acid addition salt thereof; for the manufacture of a medicament for the treatment of acute pain in humans characterised in that the medicament is for administration to an adult human in a single dose comprising from 100 to 600mg, preferably from 200 to 500mg, advantageously not more than 400mg (expressed as the weight of the free acid) of ibuprofen or a pharmaceutically acceptable salt thereof, and from 12 to 40mg, preferably from 15 to 22.5mg, advantageously not more than 20mg (expressed as the weight of the free base) of codeine or a pharmaceutically acceptable acid addition salt thereof.

Advantageously an effective amount of the medicament will be administered as a unit dose. Preferably a unit dose for adult humans comprises 400mg of ibuprofen (or an equivalent amount of a pharmaceutically acceptable salt) and 20mg of codeine (or an equivalent amount of a pharmaceutically acceptable acid addition salt). Regulations in the United Kingdom allow up to 20mg codeine and up to 400mg ibuprofen per unit dose to be incorporated into an OTC medicine.

Examples of acute pain against which medicaments of the invention are particularly effective include headaches and dental pain, where fast relief of pain is desirable.

Suitable pharmaceutically acceptable salts of ibuprofen include the sodium salt.

Suitable pharmaceutically acceptable salts of codeine include codeine acetate, codeine hydrobromide, codeine hydrochloride, codeine phosphate, codeine salicylate and codeine sulphate.

Medicaments of the invention are prepared by admixture of the appropriate ingredients, in the required amounts, and are suitably adapted for oral administration and as such may be in the form of tablets, capsules, oral liquid preparations, powders, granules, lozenges or reconstitutable powders.

2

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use in compositions of the invention include cellulose, mannitol, lactose and other similar agents. Suitable disintegrants include starch, polyvinylpolypyrrolidone and starch derivatives such as sodium starch glycollate. Suitable lubricants include, for example, magnesium stearate. Suitable pharmaceutically acceptable wetting agents include sodium lauryl sulphate.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral formulations may be prepared by conventional methods of blending, filling or tabletting. Repeated blending operations may be used to distribute the active agent throughout those compositions employing large quantities of fillers. Such operations are, of course, conventional in the art.

The present invention also provides a method of treatment of acute pain in humans, which method comprises the administration to an adult patient in need thereof of a single dose of a medicament comprising from 100 to 600mg, preferably from 200 to 500mg, advantageously not more than 400mg (expressed as the weight of the free acid) of ibuprofen or a pharmaceutically acceptable salt thereof and from 12 to 40mg, preferably from 15 to 22.5mg, advantageously not more than 20mg (expressed as the weight of the free base) of codeine, or a pharmaceutically acceptable acid addition salt thereof. Such treatment may be administered in the manner as hereinbefore described.

The present invention further provides a unit dose pharmaceutical composition for use in the single dose treatment of acute pain which comprises from 100 to 600mg, preferably from 200 to 500mg, advantageously not more than 400mg (expressed as the weight of the free acid) of ibuprofen or a pharmaceutically acceptable salt thereof, and from 12 to 22.5mg, preferably from 15 to 22.5mg, advantageously not more than 20mg (expressed as the weight of the free base) of codeine or a pharmaceutically acceptable acid addition salt thereof, and a pharmaceutically acceptable carrier in a unit dose, for adult humans. Preferably a unit dose for adult humans of the pharmaceutical composition comprises the equivalent of 400mg of ibuprofen and 20mg of codeine. Such compositions may be prepared in the manner as hereinbefore described for the medicaments.

The following formulations and clinical test results illustrate the invention.

Examples of tablets comprising ibuprofen (200mg) and codeine phosphate (13.27mg) [equivalent to 10mg codeine free base]. 2 Tablets make up a unit dose for an adult human.

| Example 1 | |
|---|---|
| | mg/tablet |
| Ibuprofen | 200.0 |
| Codeine phosphate | 13.27 |
| Maize Starch | 50.0 |
| Microcrystalline cellulose | 40.0 |
| Pre-gelatinised starch | 100.0 |
| Talc | 5.0 |
| Sodium starch glycollate | 7.5 |
| Fumed silica | 1.3 |
| Stearic acid | 6.7 |
| Lactose DT | 243.7 |

| Example 2 | |
|---|---|
| | mg/tablet |
| Ibuprofen | 200.0 |
| Codeine phosphate | 13.27 |
| Maize Starch | 70.0 |
| Microcrystalline celluose | 20.0 |
| Polyvinylpyrollidone | 10.0 |
| Talc | 5.0 |
| Sodium starch glycollate | 7.5 |
| Fumed silica | 1.3 |
| Stearic acid | 6.7 |
| Lactose | 333.7 |

Clinical Test Results

1. Clinical study comparing the analgesic activity of ibuprofen (400mg) plus codeine (20mg) versus ibuprofen (400mg) alone in acute pain

A double-blind, crossover study was conducted in 25 patients undergoing surgical removal of bilateral lower third molars. Analgesic activity was assessed after a single dose of each medicament.

Medicament was dosed orally after each operation. Pain intensity was recorded immediately before dosing and at 0.5, 1, and thereafter at hourly intervals up to 12 hours post-dosing using a 100mm visual analogue scale and a four point categorical verbal rating. Pain relief index (PRIX) was derived as described by Slatis P. et al., Curr. Ther. Research, 1980, 27, 595-600.

Table 1

| Single-dosing comparison | | |
|---|---|---|
| Test Parameter: PRIX | Ibuprofen plus codeine | Ibuprofen |
| 1. Visual Analogue Pain Intensity | 375.0±34.8* | 274.1±39.4 |
| 2. Categorical Pain Intensity | 336.4±34.5* | 288.8±37.4 |

* statistically significant at p< 0.05

Table 1 shows that the analgesic effect from a single dose of ibuprofen plus codeine is significantly greater than from a single dose of ibuprofen alone.

2. Clinical study comparing the analgesic activity of ibuprofen (400mg) plus codeine (20mg) versus a combination of paracetamol (1000mg), caffeine (60mg) and codeine phosphate (16mg)**

A second similar study in dental pain was conducted. Pain intensity was assessed after a single dose of each medicament and analgesic activity was expressed as a summed pain intensity difference (SPID).

Table 2

| Single-dosing comparison | | |
|---|---|---|
| Test Parameter | Ibuprofen plus codeine | Paracetamol plus caffeine plus codeine |
| SPID | 3.9 ± 0.6* | 2.0 ± 0.5 |

* statistically significant at p< 0.05

Table 2 shows that the analgesic effect from a single dose of ibuprofen plus codeine is almost double that from a single dose of paracetamol plus caffeine plus codeine.

The above clinical data demonstrate the beneficial effect of a single dose of ibuprofen plus codeine in the treatment of acute pain.

**Claims**

1. The use of ibuprofen or a pharmaceutically acceptable salt thereof; and codeine or a pharmaceutically acceptable acid acid addition salt thereof; for the manufacture of a medicament for the treatment of acute pain in humans; characterised in that the medicament is for administration to an adult human in a single dose comprising from 100 to 600mg (expressed as the weight of the free acid) of ibuprofen or a pharmaceutically acceptable salt thereof, and from 12 to 40mg (expressed as the weight of the free base) of codeine or a pharmaceutically acceptable acid addition salt thereof.

2. The use as claimed in claim 1 wherein the acute pain is in the form of headache.

3. The use as claimed in claim 1 wherein the acute pain is in the form of dental pain.

4. The use as claimed in any one of claims 1 to 3 wherein the medicament is for oral administration.

5. The use as claimed in claim 1, 2 or 3 wherein a single dose of medicament comprises 400mg (expressed as the weight of the free acid) of ibuprofen or a pharmaceutically acceptable salt thereof, and 20mg (expressed as the weight of the free base) of codeine or a pharmaceutically acceptable acid acid addition salt thereof.

** Available from Sterling Research under the trade name Solpadiene.

EP 0 388 125 A1

6. A unit dose pharmaceutical composition for use in the single dose treatment of acute pain in adult humans, which comprises between 100 and 600mg (expressed as the weight of the free acid) of ibuprofen or a pharmaceutically acceptable salt thereof, and between 12 and 40mg (expressed as the weight of the free base) of codeine or a pharmaceutically acceptable acid addition salt thereof, and a pharmaceutically acceptable carrier.

7. A composition as claimed in claim 6 comprising 400mg (expressed as the weight of the free acid) of ibuprofen or a pharmaceutically acceptable salt thereof, and 20mg (expressed as the weight of the free base) of codeine or a pharmaceutically acceptable acid addition salt thereof.

Claims for the following Contracting State: ES

1. The use of ibuprofen or a pharmaceutically acceptable salt thereof; and codeine or a pharmaceutically acceptable acid acid addition salt thereof; for the manufacture of a medicament for the treatment of acute pain in humans; characterised in that the medicament is for administration to an adult human in a single dose comprising from 100 to 600mg (expressed as the weight of the free acid) of ibuprofen or a pharmaceutically acceptable salt thereof, and from 12 to 40mg (expressed as the weight of the free base) of codeine or a pharmaceutically acceptable acid addition salt thereof.

2. The use as claimed in claim I wherein the acute pain is in the form of headache.

3. The use as claimed in claim 1 wherein the acute pain is in the form of dental pain.

4. The use as claimed in any one of claims 1 to 3 wherein the medicament is for oral administration.

5. The use as claimed in claim 1, 2 or 3 wherein a single dose of medicament comprises 400mg (expressed as the weight of the free acid) of ibuprofen or a pharmaceutically acceptable salt thereof, and 20mg (expressed as the weight of the free base) of codeine or a pharmaceutically acceptable acid acid addition salt thereof.

6. A process for the preparation of a unit dose pharmaceutical composition for use in the single dose treatment of acute pain in adult humans, which comprises admixing from 100 to 600 parts by weight (expressed as the weight of the free acid) of ibuprofen or a pharmaceutically acceptable salt thereof, and from 12 to 40 parts by weight (expressed as the weight of the free base) of codeine or a pharmaceutically acceptable acid addition salt thereof, with a pharmaceutically acceptable carrier, and thereafter dividing the mixture into unit dose forms,each comprising from 100 to 600mg (expressed as the weight of the free acid) of ibuprofen or a pharmaceutically acceptable salt thereof and from 12 to 40 mg (expressed as the amount of the free base) of codeine or a pharmaceutically acceptable salt thereof.

7. A process as claimed in claim 6 comprising admixing 400 parts by weight (expressed as the weight of the free acid) of ibuprofen or a pharmaceutically acceptable salt thereof, and 20 parts by weight (expressed as the weight of the free base) of codeine or a pharmaceutically acceptable acid addition salt thereof, with a pharmaceutically acceptable carrier, and thereafter dividing the mixture into unit dose forms, each comprising 400mg (expressed as the weight of the free acid) of ibuprofen or a pharmaceutically acceptable salt thereof and 20 mg (expressed as the weight of the free base) of codeine or a pharmaceutically acceptable salt thereof.

6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 274 845 (THE BOOTS CO. PLC.) * Page 4, example 1 - page 5, example 4; page 1, lines 3-11 * | 1-7 | A 61 K 31/485 // (A 61 K 31/485 A 61 K 31:19 ) |
| X | CLINICAL THERAPEUTICS, vol. 7, no. 5, 1985, pages 549-554, Princeton, US; S.L. NORMAN et al.: "A double-blind comparison of a new ibuprofen-codeine phosphate combination, codeine phosphate, and placebo in the relief of postepisiotomy pain" * Page 550: "Methods" * | 1,4-7 | |
| X | CLINICAL THERAPEUTICS, vol. 7, no. 4, 1985, pages 442-447, Princeton, US; M. CATER et al.: "A double-blind comparison of the new ibuprofen-codeine phosphate combination, zomepirac, and placebo in the relief of postepisiotomy pain" * Page 443 * | 1,4-7 | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 12-06-1990 | BERTOCCHI C. |

EPO FORM 1503 03.82 (P0401)